# EUROPEAN PATENT APPLICATION

(11) **EP 1 033 576 A2**
(43) Date of publication of application: **06.09.2000**
(21) Application number: 00104786.9
(22) Date of filing: 06.03.2000
(51) Int. Cl.: G01N 33/68

(54) **Method of preparing sample for amino acid analysis and kit for analyzing the same**

(30) Priority: 04.03.1999 CZ 77099
(71) Applicant: Phenomenex, Inc., Torrance, CA 90501 (US)
(72) Inventor: Husek, Petr, Praha 4, 147 00 Czech Republic (CZ)
(74) Representative: Schmitz, Hans-Werner, Dipl.-Ing.

(57) **Abstract**

Disclosed is a method of sample processing for the analysis of free amino acids liquid samples, particularly body fluids, by gas chromatography. Amino acids in a particular solution, plus co-mixed internal standard, are loaded directly onto a bed of cation. The bed is washed with an aqueous organic (water miscible) solvent, the captured amino acids are displaced with a medium containing aqueous sodium chloride/hydroxide (or carbonate), alkanol with 1-4 carbon atoms and a pyridine type base, treated directly in the eluate with an alkyl chloroformate of 1 to 4 carbon atoms in the alkyl moiety and after transferring the derivatized compounds into an organic solvent immiscible with water, an aliquot is subjected to the gas chromatographic analysis. Additionally, a kit for this type of analysis is described.

## Description

### Background of the Invention

The present invention relates to a method of analyzing free amino acids in complex mixtures such as body fluids and an apparatus for analyzing the same.

The extreme complexity of natural proteins which are made of twenty different amino acids called essential amino acids makes their analysis very difficult. In general, the individual quantization of the components of a complex mixtures having twenty components or more is a great challenge in analytical chemistry. The interest for a fast, effective and reliable method of analysis for amino acids has not abated in the second half of the 20th century.

The methods used at different stages of instrumental development in analytical chemistry succeeded as follows. In the 1950s free amino acids were extracted from different matrixes by ion exchange using low-pressure liquid chromatography columns. After extraction they were derivalized with ninhydrin and analyzed by photometric detection. This procedure was soon automated and amino acid analyzers (AAA) were commercially available ever since. In a further development the sensitivity of this method was enhanced 2-3 fold by using fluorometric measurements instead of photometric ones. For this purpose ninhydrin was replaced with fluorescein or ophtalaldehyde as derivatization reagent. The use of these instruments is widespread due to their robustness. Two major disadvantages are associated with this method. Firstly, a laborious sample preparation procedure is required which includes a deproteinization step for any biological sample. Second, the time required for analysis is extremely long, namely 2-3 hours.

In the 1980s a new method based on gas-chromatography (GC) allowed for the analysis of amino acids in less than 30 minutes. The excellent sensitivity of a common GC detector, the Flame Ionization Detector (FID), insured limits of detection on the order of picomoles. More specific detectors used in GC lowered the detection limits to femtomoles. The GC analysis requires derivatization but not for the purpose of detection as in AAA instruments, but rather for the purpose of transforming the amino acids in volatile compounds which may be analyzed by GC. During the last three decades hundreds of procedures for effective chemical treatment of protein amino acids have been developed. Most of these procedures required multiple steps carried out in non-aqueous media and at high temperatures (above 100° C). Most of these methods were abandoned because of their elaborate and time-consuming nature.

However, the present inventor discovered about 10 years ago that the esters of chloroformic acid may be used as new universal derivatization reagents for the GC analysis of a wide range of compounds including amino acids. In the case of amino acids the amino group is alkylated while the carboxylic group is simultaneously esterified. The reaction medium may be aqueous and the procedure is fast. With this method, the deivatization became a negligible step in the overall procedure for sample preparation for GC analysis of amino acids.

With the onset of High Performance Liquid Chromatography (HPLC) in the 1970s with its wide applicability much beyond the limits of GC, the attention shifted toward this new technique in amino acid analysis as well. Unfortunately, the most common detector used in HPLC--the UV photometric detector- cannot be used for amino acid detection. Derivatization is required again, in order to render amino acids detectable by either UV photometry, by fluorometry or by electrochemical methods. The medification of amino acid molecules can be accomplished either prior to chromatographic separation or as a post-separation step. Practice showed that preseparation derivatization is more suitable. The separation is carded out on oetadecylmodified silica columns using gradient elution. A complete amino acid profile may be obtained in 60 minutes.

Various derivatization reagents and procedures were examined for the HPLC analysis of amino acids. Most of these procedures can be carried out in aqueous media in short time (within few minutes), but with considerable disadvantages Some derivatives are not stable enough and decompose during chomatographic analysis resulting in poor reproducibility. There are no universal derivatizing reagents and no suitable reagents could be identified for some amino acids. At the same time, trace amounts of contaminants may disturb the derivatization reaction.

There is no universally applicable procedure for the HPLC analysis of amino acids. HPLC is proved useful for the determinition of amino acids in non-physiological samples. In case of physiological fluids, on the other hand, due to their complex nature and high protein content a time-consuming deproteinization step is required. Nevertheless, fully automated instruments designed for the analysis of amino acids based on HPLC are commercially available from different manufacturers.

Finally, the most recent separation technique developed in the 1990s, Capillary Zone Electrophoresis (CZE) has also been explored for the analysis of amino acids, Presently, the same derivatization reactions are applied as in HPLC, but the studies do not go beyond simple samples made up of pure standards. Further developments in the field of amino acid analysis are to be expected.

In conclusion, the drawbacks in using GC or HPLC for amino acid analysis reside more in the difficulties related to sample preparation rather then the analysis itself These laborious sample preparation procedures for both GC and HPLC, and difficulties in detection in case of HPLC discourage end-users from embracing these techniques. For these reasons the classical robust amino acid analyzing instruments based on ion-exchange are most widespread in spite of their long analysis time.

### Summary of the Invention

One aspect of the present invention relates to a method of analyzing at least one type of free amino acids contained in a fluid sample. According to the method, the fluid is ion-exchanged to retain the free amino acids onto an ion exchange resin while avoiding proteins, if any, being retained. The amino acids are released from the ion exchange resin by treating the resin with an eluting medium, thereafter the amino acids being contained In an eluate. The amino acids contained in the eluate are derivatized by adding derivatizing reagent to the eluate, in which the eluting medium serves as the derivatization reaction medium as well. The resulting amino acid derivatives are separated by chromatographic analysis.

Another aspect of the present invention relates to a kit for use in analyzing at least one type of free amino acid contained in a fluid sample. The kit comprises a sorbent cartridge filled with an ion-exchange resin for capturing amino acids when in contact with the fluid sample; an eluting medium for releasing the amino acids from the ion-exchange resin; and a derivatizing agent for derivatizing the released amino acids, the eluting medium being a reaction medium of the derivatization. The kit may further comprise a sorbent cartridge with an interior cavity, an opening at a distal end of the cavity and a porous barrier placed in the cavity. The kit may still further comprise a washing medium, an extraction medium, a chromatographic column, and amino acid standards and a solution of internal standard used in quantitative determination.

### Brief Description of the Drawings

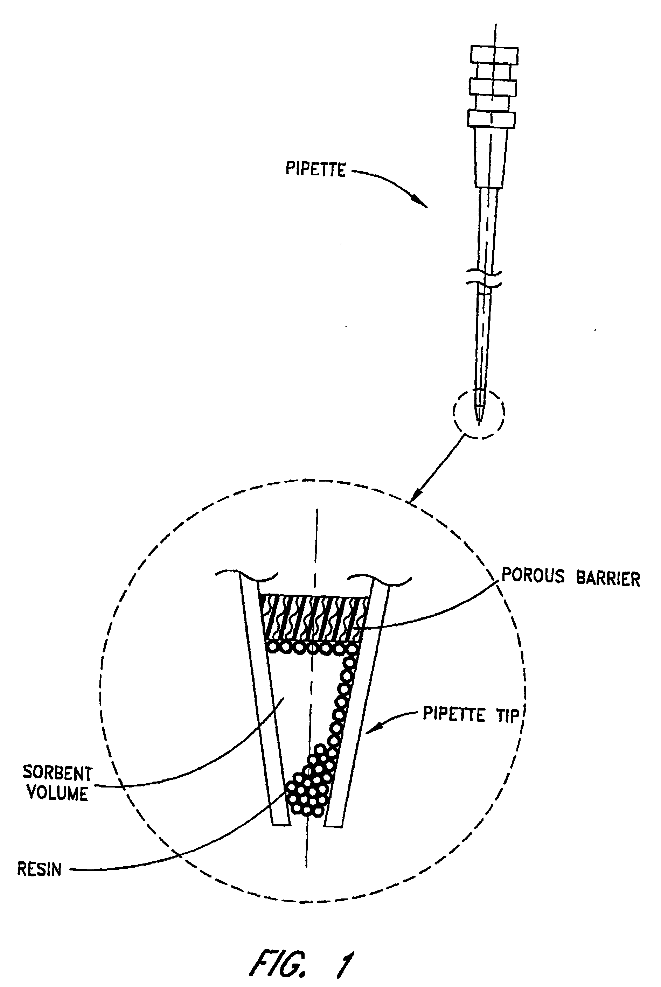

Figure 1 shows a schematic view of a sorbent cartridge.

### Detailed Description of the Embodiments of Invention

Now the various aspects of the present invention will be discussed in more detail. It is to be understood at the outset of the description which follows that persons of skill in the appropriate arts may modify the invention here described while still achieving the favorable results of this invention. Accordingly, the following description is to be understood as being a broad, teaching disclosure directed to persons of skill in the appropriate arts, and not as limiting upon the present invention.

One aspect of the present invention relates to analyzing free amino acid in a fluid sample. Complex biological fluid such as undeproteinized serum, urine or other mixtures containing amino acids can be easily prepared for analysis. Biological samples in original state, at native pH and without any pretreatment or after dilution with an aqueous solution of sodium metabisulfite (Na₂S₂O₅) or dithiothreitol is subjected to ion exchange for amino acid isolation and subsequent derivatization. The dilution may be required in scram homocysteine analysis and the dilution ratio may be as high as 1:1 (v/v).

The choice of sorbent material for retaining amino acids depends upon the chemical stability of the sorbent material under basic conditions. Silicagel based sorbents seem to be less suitable since their applicability is generally limited to a pH range of 2-8. Sorbents based on styrene-divinylbenzene copolymers with 2, 4 or 8% cross-linking are more resistant in basic media. A higher degree of cross-linking reduces particle swelling, but at the same time increases the retaining capacity of the resin for aromatic amino acids such as phenylalanine, tyrosine and tryptophane. For this reason their use is not recommended since the recovery of the aromatic amino acids in the extraction step is poor (down to 60% with 8% cross-linked resin).

However, this disadvantage can be overcome by performing the subsequent derivatization reaction in the presence of the ion exchange sorbent resin. Advantageously, after the amino acids are captured by the ion exchange sorbent, the sorbent is transferred into a reaction vessel for derivatization reaction. The derivatization reaction is performed in the presence of the ion exchanger sorbent. The outcome of the reaction is not negatively influenced by the presence of to resin in the reaction medium.

The ion exchange capacity of the cation exchange resin is to be carefully considered for a positive outcome. The Dowex 50W type resin has an ion exchange capacity of 5.2 µeq/mg. Experimental data showed that for a sample of 100 µl serum the required volume of ion exchange resin depends on resin type. 100 µl serum contain besides amino acids, also 15 µmol Na⁺ which compete for the ion exchange sites on the resin. This amount of ions requires a 1.2-1.5 fold ion exchange capacity, or 4-5 mg resin. Taking into account the degree of swelling for each type of ion exchange resin, for both 100/200 and 200/400 mesh size, the required Volume is 12, 20 and 35µl of 8, 4 and 2% cross-linked resin, respectively. The capacity of the ion exchanger of the Dowex type is high. Commercially available columns filled with ion exchange materials of other kind are delivered with 30 and more milligrams of filling, which is unnecessarily high, as according to the present invention only 4-5 mg of Dowex 50W exchanger would suffice.

In preparing a resin bed with such a small amount *in situ* within a few seconds, a preferred embodiment of the present invention: uses in a sorbent cartridge. The sorbent cartridge has a longitudinal interior cavity with a tapered tip and an opening at a distal end of the tip. A porous barrier is placed in the cavity to define a sorbent volume between the barrier, the cavity walls and the opening at the distal end of the tip. A sorbent is placed in the sorbent volume, and a fluid to be analyzed is sucked into the sorbent through the opening.

The sorbent cartridge can be formed using a laboratory pipette tip, equipped with a PE-filter as the porous barrier, available in different volumes under the filter - usually 12 µl and up. By connecting another tip with a piston (so called setter or stepper) to the pipette-tip, a slurry of the resin in an appropriate solvent can sucked into the free space (sorbent volume) under the filter until the volume is filled with sorbent, thereby forming a precisely defined volume of the exchanger bed. The solvent is sucked through the PE-filter while the sorbent stays in the sorbent volume. Preferably, a slurry made of ion exchange resin and glycerol-water 1:1 is to be used.

A fluid sample containing free amino acids is then sucked into the sorbent volume in the same way and interacts with the resin. As the sample fluid passes through the sorbent, the amino acids along with some proteins and other components of the sample are captured by the resin while the other components in the fluid sample pass through the barrier. For example, a body fluid (50 to 100 µl of plasma or serum or 100-200 µl of urine diluted with a solution of norvalin used as internal standard), followed by the washing solution, is sucked through the bed by slowly moving the piston of the setter upward. Any sample components not retained by the cation exchange resin are sucked away. During the subsequent washing, most proteins and sample components trapped in the interparticle spaces of the sorbent layer are removed and wasted. The washing solvent mix may contain any water miscible organic solvent such as isopropanol, acetonitrile or acetone, preferably 200 µl of water:n-propanol 2:1 (v/v). Thereafter, most wash liquid is removed from the ion exchange bed by sucking air into the sorbent cartridge. The setter content is discarded and to elution medium (ca. 200 to 300 µl solvent mix according to the type and volume of the ion exchanger) is sucked through the exchanger bed.

The amino acids retained on the sorbent are extracted from the sorbent cartridge with an extracting solvent mixture, which serves also as reaction medium in the subsequent derivatization step. Alternatively, the exchanger bed is pushed out together with the elution medium into the reaction vessel and the derivatization is performed in the presence of the resin, without any negative impact onto derivatization yields. The latter procedure may be used advantageously when an ion exchanger with 8% cross-linking is employed for the isolation of amino acids from to sample matrix and the aromatic amino acids are retained more strongly on the resin.

The extraction solvent/reaction medium is a mixture of water or saline solution (aqueous 0.15M NaCl), a straight or branched chained alkanol and a base. Alkanols with 1-4 carbon atoms may be used. A suitable base is pyridine or it's methyl derivatives called picolines. This extraction solvent mixture may not be strong enough to release the basic amino acids, such as lysine, ornithine, histidine and arginine from the ion exchange resin. Therefore, an addition of 0.1-3% (w/w) of a strong base like the hydroxide or carbonate of an alkali metal may enable thir released. The addition of base does not interfere with the subsequent derivatization reaction. It increases the yield for basic amino acids and for glutamic acid as well. The latter prefers hydroxide to carbonate for higher yield. In conclusion, a mixture of 1% NaOH in saline solution should be mixed with a mix of n-Propanol:3-Picoline (5:1, v/v) as an extraction solvent/reaction medium. This solution should be prepared daily by mixing the NaOH solution with the organic solvent mix in the ratio 3:2 (v/v). The final composition of the extraction solvent/reaction medium will be NaOH in saline:n-Propanol:3-picoline 9:5:1 (v/v/v).

The extract is transferred into a reaction vessel for derivatization. In order to use GC, amino acids have to be chemically transformed into volatile derivatives. The derivatization reaction is based on the concomitant alkylation of the amino group and esterification of the carboxylic group in the amino acid molecule. Alkylesters of chloroformic acid are used as derivatization reagents. The alkyl chains advantageously have 1-4 carbon atoms. Ethyl, propyl, and iso-butyl chloroformates are preferred. The ragent should be added in portions at intervals of minimum 1 min.

The amino acids are extracted from the ion exchange resin in an aqueous solvent mixture as described earlier. In contrast, the derivatization reagent is mixed into a mixture of non-aqueous solvents like iso-octane and chloroform, dichloromethane and acetonitrile, or iso-octane and dichloroethane. The resulting amino acid derivatives concentrate Into this mixture of organic solvents and may be readily analized after only one final step of sample preparation. Preferred mixtures for derivatization include acetonitrile-dichloromethane-propyl chloroformate in the ratio 3:3:2; acetonitrile-dichloromethane-ethyl chloroformate in the ratio 3:3:2 and acetonitrile-dichloromethane-propyl chloroformate in the ratio 1:1:1. The derivatization is commonly carried out at room temperature. Other organic solvents such as toluene, tert-butylmethyleter, or the latter mixed with dichloromethane may be used as extraction medium.

The alkylation-esterification of amino acids and the concomitant extraction of derivatives into the organic phase proceeds at the aqueous-nonaqueous interface. The interaction between the amino acid extract and the derivatization reagent is enhanced by vigorous mixing. Advantageously, the reagent is added in two or three consecutive portions. Between each reagent addition vigorous mixing is required. The second addition of reagent should follow the first one after 2 minutes. The third addition may follow one minute after the second one. The reaction proceeds at room temperature. Preferably, the following optimal procedure should be used; the first addition of reagent should be 50 µl of iso-octane:propyl-chloroformate 5:1 (v/v) followed by two vigorous mixing steps 2-3 seconds long each, 1 minute apart, followed by a second addition of 100µl of a mix of dichloromethane:iso-octane:propyl-chloroformate 24:16:1 (v/v/v) followed by mixing for 2-3 seconds.

Pyridine extracts together with the amino acid derivatives into the organic phase. It may interfere with the gas-chromatographic analysis and may be removed by extraction with a 1M solution of HCl. 100 µl of this solution should be added 30 seconds after the last mixing with reagent During this operation, the volume of organic layer decreases due to the concomitant extraction of propanol and pyridine in the aqueous phase. The remaining organic phase contains the amino acid derivatives and may be readily analyzed by GC. The resulting organic phase may be cloudy because of residual moisture. After removing the aqueous HCl phase it can be dried by adding some dry sodium sulfate.

The organic aliquot is analyzed by gas chromatography (GC) although not limited thereto. The analysis is performed on a medium polarity fused silica capillary column with an I.D. of 0.18-0.32mm and a length of 5-15m. Advantageously, the Zebron Amino Acid column having an I.D. of 0.25mm, a length of 10-12m and a film thickness of 0.25µm may be used. Temperature programming at a rate of 25-40° C/min insures the elution of all essential amino acids in less than 8 min.

One of the major advantages of the present process includes speed and simplicity in the sample preparation procedure and the analysis of the prepared sample. The time-consuming deproteinization and sample evaporation required before derivatization in other procedures are avoided. A complete amino acid profile may be determined in a very short time by using gas chromatography. This procedure is a succession of simple laboratory operations such as liquid dispensing, drawing liquids in a syringe, mixing liquids and separating two non-miscible phases.

Another major advantage is the excellent derivatization reaction it is based on. This reaction is applicable for any amino acid, and the derivatives are easily detected by a common universal GC detector (FID). The reaction is conducted at room temperature, proceeds in one step, it is fast and the derivatives are readily extracted in the organic phase at the end of the derivatization step. This phase is injected onto the GC for analysis. The analysis may be conducted at a later time given appropriate storage conditions.

Moreover, this process of preparation and analysis may be applied to a wide variety of samples such as body fluids, protein hydrolysates, fermentation broth, etc. Comparison studies with the existing classical AAA profiles showed good reproducibility and precision of the resulting amino acid profiles in accordance with the present method.

Another aspect of the present invention relates to providing an apparatus and a kit analyzing free amino acids in a fluid, which uses the advantageous method as discussed above. The apparatus prepares an amino acid sample including amino acids for chromatographic analysis with minimal manual operation. The apparatus advantageously may include chromatography, preferably GC. The kit advantageously includes a tool for isolating amino acids and reagents for washing, extraction and derivatization. The sorbent cartridge discussed above is preferably used as the tool for amino acid isolation from a sample fluid although not limited thereto. The reagents for washing, extraction and derivatization are preferably selected from those discussed above. A column for profiling the derivatized amino acids can also be included. The column is advantageously for a use in gas chromatography and preferably an Amino Acid Zebron fused silica capillary column. Additionally, amino acid standards and a solution of internal standard can be further included to allow for the identification and quantitation of any amino acid present in the sample. Preferably, the following standard solutions are provided:
I. 17 essential amino acids common in protein hydrolysates dissolved in 0.1M HCl
II. Asparagine, glutamine and tryptophane dissolved in neutral solution
III. Non-essential amino acids commonly found in urine

The components of the amino acid analysis kit enables a simple, fast and reproducible sample preparation for complex mixtures containing amino acids. The kit provides all the advantages discussed above in terms of the method of sample preparation and sample analysis. Also, the kit particularly has the advantage in relation to the sorbent cartridge. The sorbent cartridge used for the isolation of amino acids in complex samples is made of commercially available pipette tips holding a minimum amount of sorbent material. These cartridges are highly cost effective and their use insures further savings in reagents and waste in the subsequent steps of sample preparation.

Various features of the present invention will be further discussed in terms of the following example, which is intended to illustrate the present invention but not limit the scope of the present invention. The necessary solvents and reagents used in the following examples were obtained from Sigma-Aldrich-Fluka.

### Example 1

A 100 µl sample of serum is diluted with an internal standard solution in the ratio 2:1. Norvalin may be used as an internal standard in a concentration of 400 µmol/l dissolved into 0.2% potassium oxalate (K₂C₂O₄) +0.5% sodium metabisulfite (Na₂S₂O₅). The serum sample is sucked into the sorbent cartridge. The sorbent cartridge is attached to a setter, a small volume syringe with a plunger. By drawing back the plunger slowly, vacuum is created in the setter volume which is in liquid connection with the sorbent cartridge. The sample cartridge is filled with BioRad AG 50Wx2 cation exchange resin (100/200 mesh) In Biotech grade. The amino acid components of the serum sample are retained onto the ion exchange resin. In the next step, 200 µl of wash solution is passed through the sobent bed. The piston is pulled slowly back until all the liquid is collected in the setter volume. The liquid is discarded. Next, the amino acids are extracted from the sorbent bed with 200 µl extraction solvent/reaction medium. The piston should be withdrawn in a slow movement until all extracting solvent is evacuated from the sorbent bed and accumulates in the setter volume. The extract is transferred into a reaction vial. Next the extractive alkylation solution is added into the reaction vial in three portions 50 µl each. Vigorous mixing is required after each addition for about 3 seconds. One minute after the third addition followed by vortexing, add 100 µl of 1M HCl solution and vortex for 10-15s. The organic layer containing the amino acid derivatives may be injected into the gas chromatograph. A complete profile will result in less than 8 minutes.

The following table summarizes results obtained from 8 measurements of the sane serum blend. Statistical evaluation was done according to Horn (J. Horn; J. Amer. Statistic Assoc., vol. 78 (1983)).

| | P_{L} nmol/mL | C.V. | | P_{L} Nmol/mL | C.V. |
|---|---|---|---|---|---|
| GLY | 402 | 2.8 | SER | 205 | 8.2 |
| ALA | 545 | 3.7 | HYP | 11 | 15.7 |
| ABA | 17 | 5.0 | ASN | 71 | 4.3 |
| VAL | 251 | 3.7 | GLN | 599 | 4.3 |
| LEU | 141 | 6.3 | CYS | 4 | 12.2 |
| ILE | 66 | 4.5 | PHE | 79 | 5.3 |
| MET | 37 | 7.9 | TYR | 60 | 6.9 |
| PRO | 206 | 3.2 | TRP | 49 | 6.8 |
| ASP | 53 | 5.0 | ORN | 138 | 7.8 |
| GLU | 181 | 9.2 | LYS | 214 | 6.1 |
| THR | 170 | 5.0 | HIS | 84 | 11.4 |

- Legend to the table:: P_{L} ... mean (according to Horn)
C.V. ... coefficient of variation

### Example 2

Diluted serum to that in Example 1 was spiked with amino acid standards having a concentration of 200 nmol/mL each in order to examine recoveries, i.e., the reproducibility and precision of the determination.

The results obtained are shown in the following table (mean values for 8 analyses; according to Horn):

| | P_{L} Nmol/mL | C.V. | Recovery (%) | | | P_{L} nmol/mL | C.V. | Recovery (%) |
|---|---|---|---|---|---|---|---|---|
| GLY | 611 | 1.5 | 105 (101-108) | | SE R | 451 | 8.1 | 122 (104-140) |
| ALA | 752 | 3.7 | 102 (91-113) | | HY P | 212 | 4.3 | 101 (95-107) |
| ABA | 219 | 4.2 | 101 (94-108) | | AS N | 270 | 5.3 | 99 (92-106) |
| VAL | 464 | 5.5 | 106 (80-131) | | GL N | 808 | 4.2 | 103 (84-122) |
| LEU | 335 | 4.5 | 97 (78-115) | | CY S | 221 | 5.8 | 108 (102-114) |
| ILE | 254 | 4.6 | 94 (84-104) | | PH E | 282 | 6.4 | 101 (86-117) |
| MET | 238 | 2.7 | 100 (95-106) | | TY R | 248 | 4.3 | 93 (84-103) |
| PRO | 421 | 7.0 | 109 (82-136) | | TR P | 250 | 8.3 | 101 (88-114) |
| ASP | 247 | 5.9 | 97 (90-104) | | OR N | 339 | 5.1 | 94 (85-103) |
| GLU | 368 | 6.7 | 96 (81-111) | | LY S | 404 | 4.4 | 94 (85-103) |
| THR | 397 | 8.9 | 111 (87-135) | | HIS | 282 | 4.0 | 100 (86-113) |

- Legend to the table:: P_{L} ... mean (according to Horn)
C.V. ... coefficient of variation

### Example 3

Plasma samples were subjected to analysis according to the present invention using gas chromatography as described in Example 1 and the results were compared with those obtained with the automated Amino Acid Analyzer with ninhydrine detection (IEC, analyzer manufactured by Mikrotechna, Czech Rep. using the procedure described by Hans. J. Bremer et al.; Disturbances of amino acid metabolism: clinical chemisty and diagnosis; Urgan & Schwarzenberg, Baltimore-Muenchen, 1981).

The following results were obtained (in nmol/ml, statistical evaluation according to Horn):

| | | | | | | | | |
|---|---|---|---|---|---|---|---|---|
| P_{L} | 270.0 | 230.5 | 530.5 | 471.5 | 29.0 | 20.0 | 316.5 | 281.5 |
| L_{L} | 265.8 | 194.9 | 515.8 | 406.6 | 24.8 | 7.4 | 272.5 | 254.3 |
| L_{U} | 274.2 | 266.1 | 545.2 | 536.4 | 33.2 | 32.6 | 360.5 | 308.7 |
| | GLY | | ALA | | ABA | | VAL | |
| | IEC | GC | IEC | GC | IEC | GC | IEC | GC |
| | 297 | 222 | 645 | 453 | 28 | 17 | 330 | 275 |
| | 270 | 264 | 529 | 487 | 32 | 16 | 307 | 315 |
| | 271 | 218 | 534 | 456 | 25 | 23 | 327 | 288 |
| | 257 | 239 | 511 | 505 | 30 | 19 | 288 | 280 |
| | 269 | 235 | 527 | 468 | 30 | 23 | 306 | 270 |

| | | | | | | | | |
|---|---|---|---|---|---|---|---|---|
| P_{L} | 167.5 | 152.5 | 77.5 | 75.5 | 44.5 | 39.0 | 197.0 | 165.0 |
| L_{L} | 119.3 | 104.3 | 54.5 | 44.1 | 38.2 | 30.6 | 37.9 | 160.8 |
| L_{U} | 215.7 | 200.7 | 100.5 | 106.9 | 50.8 | 47.4 | 356.1 | 169.2 |
| | LEU | | ILE | | MET | | PRO | |
| | IEC | GC | IEC | GC | IEC | GC | IEC | GC |
| | 156 | 132 | 62 | 67 | 41 | 37 | 235 | 164 |
| | 224 | 173 | 72 | 87 | 46 | 43 | 241 | 186 |
| | 159 | 164 | 81 | 83 | 47 | 39 | 159 | 166 |
| | 151 | 141 | 83 | 70 | 43 | 41 | 151 | 164 |
| | 179 | 143 | 90 | 68 | 44 | 31 | 228 | 165 |

| | | | | | | | | |
|---|---|---|---|---|---|---|---|---|
| P_{L} | 175.0 | 163.5 | 117.5 | 91.0 | 33.5 | 17.0 | 147.5 | 142.0 |
| L_{L} | 145.7 | 136.3 | 94.5 | 70.1 | 10.5 | 4.4 | 91.0 | 116.9 |
| L_{U} | 204.3 | 190.7 | 140.5 | 111.9 | 56.5 | 29.6 | 204.0 | 167.1 |
| | THR | | SER | | ASP | | GLU | |
| | IEC | GC | IEC | GC | IEC | GC | IEC | GC |
| | 209 | 146 | 123 | 81 | 43 | 14 | 114 | 136 |
| | 179 | 165 | 119 | 107 | 35 | 14 | 163 | 128 |
| | 182 | 170 | 123 | 96 | 39 | 24 | 161 | 148 |
| | 168 | 157 | 112 | 86 | 28 | 18 | 152 | 137 |
| | 159 | 179 | 99 | 94 | 19 | 20 | 134 | 157 |

| | | | | | | | | |
|---|---|---|---|---|---|---|---|---|
| P_{L} | 622.5 | 575 | 28.0 | 23.5 | 76.5 | 74.0 | 104.0 | 97.5 |
| L_{L} | 566.0 | 453.5 | 23.8 | 17.2 | 61.8 | 69.8 | 9134 | 82.8 |
| L_{U} | 679.0 | 696.5 | 32.2 | 29.8 | 91.2 | 78.2 | 116.6 | 112.2 |
| | GLN | | CYS₂ | | PHE | | TYR | |
| | IEC | GC | IEC | GC | IEC | GC | IEC | GC |
| | 739 | 493 | 44 | 23 | 84 | 83 | 82 | 94 |
| | 636 | 563 | 29 | 25 | 73 | 74 | 107 | 101 |
| | 636 | 604 | 21 | 31 | 76 | 73 | 106 | 107 |
| | 609 | 546 | 27 | 22 | 80 | 66 | 108 | 91 |
| | 596 | 624 | 27 | 20 | 66 | 75 | 101 | 96 |

| | | | | | | | | |
|---|---|---|---|---|---|---|---|---|
| P_{L} | - | 57.5 | 87.0 | 58.0 | 185.0 | 153.5 | 118.0 | 106.0 |
| L_{L} | - | 34.5 | 74.4 | 37.1 | 159.9 | 130.5 | 101.2 | 59.9 |
| L_{U} | - | 80.5 | 99.6 | 78.9 | 210.1 | 176.5 | 134.8 | 152.1 |
| | TRP | | ORN | | LYS | | HIS | |
| | IEC | GC | IEC | GC | IEC | GC | IEC | GC |
| | | 63 | 81 | 53 | 198 | 159 | 114 | 92 |
| | | 52 | 89 | 78 | 182 | 167 | 122 | 118 |
| | | 57 | 100 | 57 | 191 | 158 | 127 | 112 |
| | | 67 | 84 | 51 | 170 | 144 | 114 | 95 |
| | | 41 | 90 | 63 | 179 | 148 | 115 | 117 |

- Legend to the table:: P_{L} ... mean (according to Horn)
L_{L} ... lower limit
L_{U} ... upper limit
IEC - measurement on the automated amino acid analyzer
GC - measurement by the method according to the invention

Comparison of the results achieved by the method according to to present invention and by the conventional automated amino acid analyzer showed that the new procedure yields equally valid data, along with substantial reduction of total analysis time and cost.

Although this invention has been described in terms of a certain preferred embodiment, other embodiments apparent to those of ordinary skill in the art are also within the scope of this invention. For instance, as will be readily apparent to those skilled in the art, however, certain aspects of each embodiment can be combined with other embodiments. Accordingly, the scope of the invention is intended to be defined only by the claims that follow.

## Claims

1. A method of analyzing at least one type of free amino acid contained in a fluid sample, the method comprising:
retaining the free amino acids contained in a liquid sample onto an ion exchange resin while rejecting proteins as much as possible;
releasing the amino acids from the ion exchange resin with an eluting medium, thereby the amino acids being contained in an eluate;
derivatizing the amino acids by adding derivatizing agent to the elute, the eluting medium being a reaction medium of the derivatization; and
separating the amino acid derivatives from a resulting mixture of the derivatization by gas-chromatography.

2. The method as defined in Claim 1, wherein the derivatization is alkylation of amino and esterification of the carboxyl group of the amino acid with an derivatizing agent.

3. The method as defined in Claim 1, wherein the releasing and derivatizing the amino acids may be preformed concomitantly in the presence of the resin.

4. The method as defined in Claim 2, wherein the eluting medium is water miscible basic solvent.

5. The method as defined in Claim 4, wherein the extraction solvent is water miscible while the derivatization reagent is added in a non-aqueous solvent The amino add derivatives extract into the non-aqueous phase.

6. The method as defined in Claim 2, wherein the alkylating/esterifying reagent comprises an alkyl chloroformate.

7. The method as defined In Claim 6, wherein the alkylating agent comprises alkyl chloroformate containing 1-4 carbon atoms in the alkyl moiety.

8. The method as defined in Claim 7, wherein the alkylating agent comprises ethyl-, propyl- or iso-butyl-chloroformate.

9. The method as defined in Claim 2, wherein the alkylation is performed in the presence of the ion exchange resin.

10. The method as defined in Claim 1, wherein the ion-exchange is performed with a styrene-divinylbenzene cation exchanger.

11. The method as defined in Claim 1, wherein the ion exchange resin retaining the amino acids is washed with a solvent comprising a water miscible alkanol.

12. The method as defined in Claim 10, wherein the water miscible alkanol comprises ethanol or propanol.

13. The method as defined in Claim 1, wherein the ion-exchanging step comprises:
placing the ion-exchange resin in an interior cavity of a sorbent cartridge defined between an opening at a distal end of the cavity, cavity walls and a porous barrier placed in the cavity; end
sucking the fluid into the cavity through the opening such that the fluid contacts with the ion-exchange resin and free amino acids in the fluid are captured by the resin.

14. The method as defined in Claim 13, wherein the ion-exchange resin is placed in the cavity by sucking a slurry of the resin suspended in a solvent through the opening.

15. The method as defined in Claim 13, wherein a material for the porous barrier is selected such that the barrier allows the fluid to pass there through but prevents passage of the resin.

16. The method as defined in Claim 15, wherein location of the porous barrier in the cavity defines a volume of the resin placed in the interior cavity of the sorbent cartridge.

17. The method as defined in Claim 15, wherein the porous barrier can be relocated so tat the volume of the resin can vary.

18. The method as defined in Claim 1, further comprising analyzing the amino acid derivatives using chromatography.

19. The method as defined in Claim 18, wherein the analysis uses gas chromatography.

20. The method as defined in Claim 1, wherein the sample is a biological fluid.

21. A kit for use in analyzing at least one kind of free amino acids contained in a fluid, the kit comprising:
an ion-exchange resin for capturing amino acids when contacting with the fluid;
an eluting medium for releasing the amino acids from the ion-exchange resin; and
a derivatizing agent for derivatizing the released amino acids, the eluting medium being a reaction medium of the derivatization.

22. The kit as defined in Chum 21, further comprising a sorbent cartridge with an interior cavity, an opening at a distal end of the cavity and a porous barrier placed in the cavity.

23. The kit as defined in Claim 22, wherein the ion-exchange resin is in a slurry form.

24. The kit as defined in Claim 22, wherein the ion-exchange resin is loaded into the cavity of the sorbent cartridge.

25. The kit as defined in Claim 22, wherein the sorbent cartridge comprises a pipette tip with a filter.

26. The kit as defined in Claim 23, further comprising a stepper with a piston.

27. The kit as defined in Claim 21, further comprising a washing medium for washing the ion-exchange resin after the amino acids are captured.

28. The kit as defined in Claim 21, wherein the the eluting medium is water miscible basic solvent.

29. The kit as defined in Claim 21, further comprising an extraction medium for extracting the amino acid derivatives from a derivatization resulting mixture.

30. The kit as defined in Claim 21, further comprising a chromatographic column for profiling the derivatized amino acids.

31. The kit as defined in Claim 30, wherein the column comprises a fused silica capillary column.

32. The kit as defined in Claim 21, further comprising amino acid standards and/or a solution of internal standard for the identification and quantitation of amino acids.
